# EUROPEAN PATENT SPECIFICATION

(11) **EP 3 971 776 B1**
(45) Date of publication and mention of the grant of the patent: **25.11.2026**
(21) Application number: 20306050.4
(22) Date of filing: 18.09.2020
(51) Int. Cl.: G06K 19/077, A61M 5/00, G06M 1/00, G16H 20/17

(54) **MEDICAL CONTAINER COMPRISING AN RFID TAG FOR REMOTE IDENTIFICATION**
MEDIZINISCHER BEHÄLTER MIT RFID-ETIKETT ZUR FERNIDENTIFIZIERUNG DES MEDIZINISCHEN BEHÄLTERS
RÉCIPIENT MÉDICAL COMPRENANT UNE ÉTIQUETTE RFID POUR L'IDENTIFICATION À DISTANCE DUDIT RÉCIPIENT MÉDICAL

(43) Date of publication of application: 23.03.2022
(73) Proprietor: Becton Dickinson France, 38800 Le Pont-de-Claix (FR); Becton, Dickinson and Company, Franklin Lakes, NJ 07417-1880 (US)
(72) Inventor: RIVIER, Cédric, 38340 VOREPPE (FR); EUVRARD, Nicolas, NC 27701 (US)
(74) Representative: Germain Maureau

(56) References cited:
- WO-A1-2019/189449
- WO-A1-2019/189451
- US-A1- 2006 186 204
- US-A1- 2006 232 413
- US-A1- 2017 124 264
- US-A1- 2018 093 042
- US-A1- 2021 008 275

## Description

The present invention relates to a medical container comprising a RFID tag and a method for manufacturing said medical container.

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to a medical container of the invention, and the "proximal direction" is to be understood as meaning the opposite direction to said direction of injection, that is to say the direction towards the user's hand holding a container as for an injection operation.

Medical injection devices, for example pre-fillable or prefilled syringes, usually comprise a hollow body or barrel forming a container for a medical product. This body comprises a distal end, optionally provided with a needle, and a proximal end, usually provided with a flange.

There is an increasing need for individual traceability of the medical containers, such as medical injection devices, from the manufacturing process until the final labeling, the final use or the disposal of said medical containers.

It is known, for example, from WO2017157784, a receptacle having a cylindrical lateral surface surrounded by a sequence of printed machine-readable unique identifier codes. These printed unique identifier codes allow tracking of each receptacle along a supply chain. However, these unique identifier codes are printed on an external side of the receptacle so that they may be removed or damaged, for example, during handling or use of the receptacle. Moreover, the unique identifier codes cover a portion of the receptacle so that they may have an impact on a user visual inspection process. Finally, an inkjet printer is used to print the identifier codes on the external side of the receptacle. However, this printing method, using ink, may lead to a risk of contamination of the receptacle. Moreover, one may not have access to these printed unique identifier codes when the receptacle is put, for example, in a sealed packaging.

It is known from the document WO 2019189451 a pre-filled syringe comprising a RFID tag positioned on the insertion section of the syringe plunger. The document US20060186204 discloses a combined multi-frequency electromagnetic and optical communication system. The document US20060232413 disloses a RFID tag with an antenna comprising an optical code. The document US2018009042 discloses a sensor for capacitive determination of a filling level. It is known from document WO2019189449 a prefilled syringe which includes an RFID tag.

In this context, an object of the present invention is to provide a device that alleviates the above-mentioned drawbacks by allowing an efficient individual identification of a medical container with a limited impact on visual inspection, with a limited impact on the manufacturing process and with an improved reading range.

The invention is defined by the independent claims. Preferred embodiments are set out in the dependent claims.

The medical container of the invention allows individual traceability of each medical container from the manufacturing process to the final use of the medical container. Indeed, the RFID tag comprised in the medical container of the invention allows remote identification of said medical container. The fact that the antenna forms, follows or overlays with at least a portion of the scale permits to increase the antenna length, thereby improving the reading range. Besides, there is a limited impact on product aspect, hence on visual inspection because the antenna is close to the scale, or even forms or overlays with the scale. There is also a limited impact on the product image at the end user.

By antenna forming at least a portion of the scale it should be understood that the antenna itself may be said at least one portion of the scale. Without the antenna, there is thus no such portion of the scale. It should be noted that the antenna may form the complete scale.

Alternatively, by antenna following at least a portion of the scale it is should be understood that the antenna may extend contiguous to said at least one portion of the scale. As a result, the antenna may run alongside the scale, and/or may intersect the scale, for instance at one or several graduation marks if any.

By antenna overlaying with the at least one portion of the scale it should be understood that the antenna is superjacent to said at least one portion of the scale and thus extends on the outline of the scale.

In an embodiment, the RIFD tag includes a chip, the chip and the antenna being applied on a transparent substrate. The substrate allows maintaining the chip and the antenna together, and the substrate's transparency aims at further limiting the impact on the medical container visual inspection.

In an embodiment, the substrate is provided with an adhesive for attachment of the RFID tag to the barrel.

This permits a limited impact on the manufacturing process.

In an embodiment, the chip and/or the antenna of the RFID tag are formed by ceramic metallic printing, metallic printing or graphene printing, etching or stamping.

In an advantageous embodiment, at least the antenna of the RFID tag is formed by graphene, metallic or ceramic metallic printing directly onto an external wall of the barrel. The chip of the RFID tag may also be formed by metallic printing or graphene printing.

This enables very thin chip and antenna, hence having an even more limited impact on the medical container external dimensions.

Preferably, the RFID tag is an Ultra-High Frequency RFID tag (UHF-RFID).

Preferably, the antenna is a dipole antenna comprising two legs attached to a chip of the RFID tag, each of said legs forming, following, or overlaying with the at least one portion of the scale.

Preferably, one of said legs extends proximal to the chip while the other leg extends distal to the chip.

This improves the reading range.

In an embodiment, the RFID tag includes a chip that is no wider than the antenna.

This limits the impact on visual inspection of the medical container. The chip may thus be hidden by the scale that may be formed by the antenna.

In an embodiment, the scale is a graduated scale. Also, the antenna may have anyone of a straight line, or a sine, a square, a triangle, a sawtooth or a pulse waveform.

Therefore, the antenna may form or overlay with any of the graduations of the graduated scale.

The invention and the advantages arising therefrom will clearly emerge from the detailed description that is given below with reference to the appended drawings as follows:
- Figure 1 is a side view of a medical container according an embodiment of the invention,
- Figure 2 is a perspective view of the barrel of a medical container according to an embodiment of the invention,
- Figure 3 is a schematic view of a RFID tag of a medical container according to an embodiment of the invention,
- Figure 4 is a view illustrating different possible shapes of the antenna of the RFID tag of the medical container according to an embodiment of the invention.

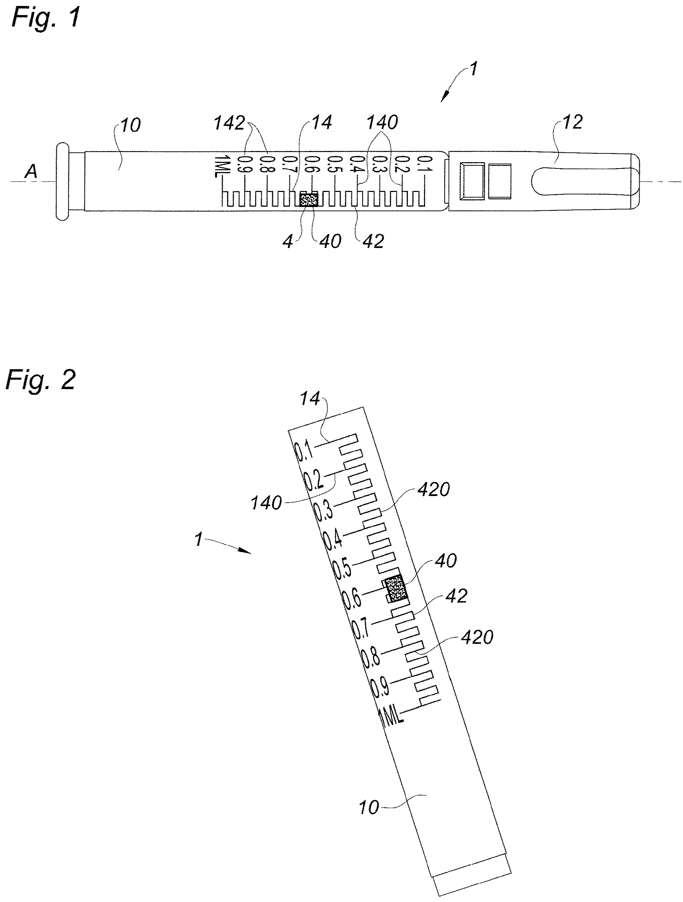

With reference to Figure 1 is shown a medical container 1, such as a pre-fillable or prefilled syringe, according to an embodiment of the invention. The medical container could also be a vial, a cartridge, or any medical container on which a volumetric scale may be implemented. The medical container 1 comprises a cylindrical barrel 10 defining a reservoir for containing a medical product. The barrel 10 extends along a longitudinal axis A and has a distal end (not shown) that may be in the form of a longitudinal distal tip defining a fluid passageway in fluid communication with the reservoir. As shown on Figure 1, a cap 12 may cover the distal tip before use of the medical container 1. The medical container 1 may also include a plunger rod (not shown) having a plunger stopper at a distal end thereof so as to expel the medical product contained in the reservoir. The barrel 10 may be made of a glass or a plastic material.

As visible on Figure 1 or 2, the barrel 10 has a graduated scale 14 extending parallel to the longitudinal axis A. The graduated scale 14 includes graduation marks 140. The graduation marks 140 may be indicative of a remaining volume of the medical product inside the reservoir or of the already injected medical product during use of the medical container 1. The graduation marks 140 may be orthogonal to the longitudinal axis A and may also be parallel to each other. A figure 142 indicative of a volume amount may be located adjacent to specific graduation line 140.

Still with reference to Figures 1 and 2, the medical container 1 further comprises a RFID tag 4, preferably a passive tag, located on the barrel 10. The RFID tag 4 is configured to allow remote identification of the medical container 1. The RFID tag 4 includes a chip 40 and an antenna 42. The chip 40 may include a memory for storing a Unique Device Identifier (UDI), and additional data such as product code or batch number.

According to the invention, the antenna 42 partially or completely forms, follows, or overlays with the graduated scale 14. In the example shown on Figures 1 and 2, the antenna 42 completely forms the graduated scale 14. That is, the antenna 42 is the graduated scale 14. In other embodiments not shown, the antenna 42 may be added to the barrel 10 such that the antenna 42 is contiguous to, or overlays with an already existing graduated scale 14. The fact that the antenna 42 itself forms, or extends along, or is superjacent with the graduated scale 14 or at least a portion of the graduated scale 14 enables to limit the visual impact on the medical container 1 appearance.

In an embodiment not shown, the medical container 1 may for instance include already existing graduation marks 140 and the antenna 42 may extend parallel to the longitudinal axis A so as to connect said graduation marks 140. In another embodiment (not shown), the antenna 42 may only form one or several graduation marks 140.

In the example of Figures 1 and 2, the antenna 42 substantially extends parallel to a longitudinal axis A of the medical container 1, and defines peaks orthogonal to said longitudinal axis A at predetermined intervals along the barrel 10. These peaks form the graduation marks 140.

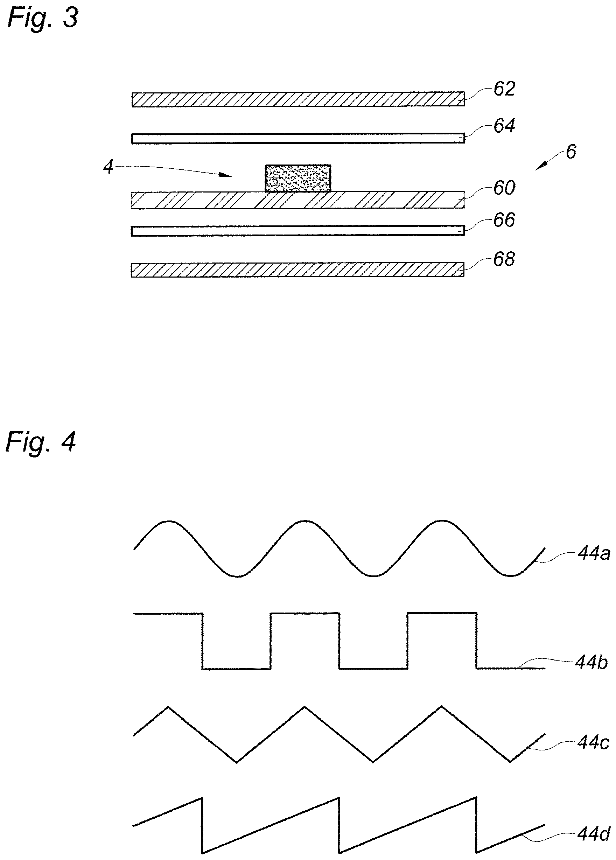

Although the antenna 42 shown on Figures 1 and 2 has a square waveform forming the graduated scale 14, it is contemplated that any other form defining graduation marks 140 along the longitudinal axis A of the barrel 10 might be appropriate. For example, as illustrated on Figure 4, the antenna 42 may have anyone of a sine 44a, a square 44b, a triangle 44c, a sawtooth 44d or a pulse waveform. Alternatively, the antenna 42 may also be shaped as a straight line extending along the longitudinal axis A, the graduation marks 140 not being overlaid or superjacent with the antenna 42. Otherwise, the graduated scale 14 may have a substantially ring shape designed around an outer wall of the barrel 10. Accordingly, the antenna 42 may follow the shape of such ring-shaped graduated scale 14.

With reference to Figure 3, the RFID tag 4 may be in a form of a transparent wet inlay 6. Alternatively, the RFID tag 4 may be in a form of a dry inlay or a pressure sensitive label. RFID Wet Inlays 6 and RFID Dry Inlays can comprise a substrate 60, for example made of paper or Polyethylene terephthalate (PET). The RFID tag 4 can be disposed on one side of the substrate 60. RFID Wet Inlays and RFID Dry Inlays can further comprise at least one protective layer 62, such as a siliconized paper, on top of the RFID tag 4. The protective layer 62 may be attached by means of an adhesive layer 64.

RFID Wet Inlays are described as "wet" as they include an adhesive backing 66 on the other side of the substrate 60 and a backing paper 68, for example a silicon liner. RFID Wet Inlays are like RFID stickers and are ideal for applications which require a "peel and stick" type of tag. RFID Dry Inlays are described as "dry" due to their lack of adhesive backing. Pressure-sensitive labels are analogous to a high-tech sticker.

It should be noted that the RFID inlay 6 is configured to resist to sterilization processes such as steam sterilization, e-beam sterilization, vapor hydrogen peroxide sterilization, or EtO sterilization.

In one embodiment, the chip 40 and/or the antenna 42 may be formed by ceramic metallic printing, metallic printing or graphene printing. They may also be formed by etching or stamping, except when they are directly formed onto the barrel 10. The ceramic metallic, metallic or graphene printing may be made on the RFID inlay 6, or directly on the external wall of the barrel 10. When the chip 40 and/or the antenna 42 are directly printed on the external wall of the barrel 10, there may be no need for a RFID inlay 6 and more specifically no need for a substrate other than the barrel itself nor for an adhesive. Preferably, at least the antenna 42 is formed by metallic printing, ceramic metallic printing or graphene printing.

The RFID tag 4 may be a Low Frequency (about 30 KHz to 300 KHz) RFID tag 4 (LF-RFID), a High Frequency (about 1-15 MHz) RFID tag 4 (HF-RFID) or, preferably, an Ultra-High Frequency (about 400-1000 MHz) RFID tag 4 (UHF-RFID). A RFID reader can for example read the LF-RFID tag 4 at a distance up to about 10 cm, the HF-RFID tag 4 at a distance of about one meter and the UHF-RFID tag 4 at a distance of about fifteen meters.

The RFID tag 4 may also be a High-Frequency Near Field Communication (HF-NFC) tag. The frequencies are usually about 13.56 MHz. In this embodiment, a NFC reader can for example read the HF-NFC tag at a distance up to a few centimeters. HF-NFC differs from HF-RFID in that it can be read by a NFC smartphone. In one embodiment, the RFID tag 4 is a double frequency tag including simultaneously a HF-NFC and an UHF RFID. For example, it can be read with both a NFC smartphone or an UHF reader.

With reference to Figure 2 where the RFID tag 4 is a UHF-RFID tag 4, the antenna 42 may be a dipole-shaped antenna 42 comprising two legs 420 attached to the chip 40. Each of said legs 420 forms, follows, or overlays with at least a portion of the graduated scale 14 or the complete graduated scale 14. Preferably, one of said legs 420 extends proximal to the chip 40 while the other leg 420 extends distal to the chip 40.

Depending on the frequencies used (UHF, HF, LF, NFC) different designs of antenna 42 are possible. For instance, when the RFID tag 4 is a HF-RFID or NFC-RFID, the antenna 42 may have a substantially ring shape and may thus form one graduation line of the graduated scale 14.

In an embodiment, the RFID tag 4 includes a chip 40 that is no wider than the antenna 42 so that the chip 40 can be hidden within the scale pattern thanks to its small size. For example the chip 40 may be about 1 mm-wide. The antenna 42 and the chip 40, usually the thickest element of the RFID tag 4, are thin enough so that they add limited extra layer to the RFID inlay, for a maximum RFID inlay thickness up to 0.5 mm, preferably below 0.4 mm, and ideally below 0.3 mm.

The invention also relates to a method for manufacturing the above-described medical container 1, the method comprising the steps of forming a RFID antenna 42 that forms, follows or overlays with at least a portion of the barrel 10 graduated scale 14.

In an embodiment, the method comprises the step of applying the antenna 42 and a chip 40 of the RFID tag 4 on a transparent substrate 60, for example by printing, etching or stamping. During this first step, the antenna 42 may be first applied onto the substrate 60 for example by printing, etching or stamping, and then the chip 40 may be added onto the substrate 60 which holds the antenna 42 and is bonded to said antenna 42. Further, the method comprises the steps of applying the substrate 60 comprising said RFID tag 4 onto an external wall of the barrel 10, preferably by means of an adhesive, and storing a Unique Device Identifier (UDI) into the RFID tag 4.

In an alternate embodiment, the method comprises the steps of applying the antenna 42 and a chip 40 of the RFID tag 4 on an external wall of the barrel 10, preferably by metallic or graphene printing, and storing a Unique Device Identifier (UDI) into the RFID tag 4.

## Claims

1. Medical container (1) comprising a tubular barrel (10) defining a reservoir for a medical product, the barrel (10) being provided with a scale (14) having graduations indicative of an injected volume or a remaining volume of said medical product, the barrel (10) further comprising a RFID tag (4) located on the barrel (10), **characterized in that** the RFID tag (4) comprises an antenna (42) that forms at least a portion of the scale (14).

2. Medical container (1) according to the preceding claim, wherein the RFID tag (4) includes a chip (40), the chip (40) and the antenna (42) being applied on a transparent substrate (60), wherein the substrate (60) is applied onto an external wall of the barrel (10).

3. Medical container (1) according to the preceding claim, wherein the substrate (60) is provided with an adhesive (66) for attachment of the RFID tag (4) to the barrel (10).

4. Medical container (1) according to claim 1, wherein at least the antenna (42) of the RFID tag (4) is formed by ceramic metallic printing, metallic printing or graphene printing directly onto an external wall of the barrel (10).

5. Medical container (1) according to any of the preceding claims, wherein the RFID tag (4) is an Ultra-High Frequency RFID tag (4).

6. Medical container (1) according to the preceding claim, wherein the antenna (42) is a dipole antenna (42) comprising two legs (420) attached to a chip (40) of the RFID tag (4), each of said legs (420) forming the at least one portion of the scale (14).

7. Medical container (1) according to the preceding claim, wherein one of said legs (420) extends proximal to the chip (40) while the other leg extends distal to the chip (40).

8. Medical container (1) according any of the preceding claims, wherein the RFID tag (4) includes a chip (40) that is no wider than the antenna (42).

9. Medical container (1) according to any of the preceding claims, wherein the antenna (42) has anyone of a straight line, or a sine, a square, a triangle, a sawtooth or a pulse waveform.

10. Method for manufacturing a medical container (1) according to any of the preceding claims, wherein the method comprises the steps of forming a RFID antenna (42) that forms at least a portion of the barrel (10) scale (14).

## Patentansprüche

1. Medizinischer Behälter (1), der einen röhrenförmigen Zylinder (10) umfasst, der ein Reservoir für ein medizinisches Produkt definiert, wobei der Zylinder (10) mit einer Skala (14) versehen ist, die Graduierungen aufweist, die ein injiziertes Volumen oder ein Restvolumen des medizinischen Produkts anzeigen, wobei der Zylinder (10) ferner einen an dem Zylinder (10) befindlichen RFID-Tag (4) umfasst, **dadurch gekennzeichnet, dass** der RFID-Tag (4) eine Antenne (42) umfasst, die mindestens einen Teil der Skala (14) bildet.

2. Medizinischer Behälter (1) nach dem vorhergehenden Anspruch, wobei der RFID-Tag (4) einen Chip (40) einschließt, wobei der Chip (40) und die Antenne (42) auf ein transparentes Substrat (60) aufgebracht werden, wobei das Substrat (60) auf eine Außenwand des Zylinders (10) aufgebracht wird.

3. Medizinischer Behälter (1) nach dem vorhergehenden Anspruch, wobei das Substrat (60) mit einem Klebstoff (66) zur Befestigung des RFID-Tags (4) am Zylinder (10) versehen ist.

4. Medizinischer Behälter (1) nach Anspruch 1, wobei mindestens die Antenne (42) des RFID-Tags (4) durch Keramik-Metall-Druck, Metall-Druck oder Graphen-Druck direkt auf eine Außenwand des Zylinders (10) gebildet wird.

5. Medizinischer Behälter (1) nach einem der vorhergehenden Ansprüche, wobei der RFID-Tag (4) ein Ultrahochfrequenz-RFID-Tag (4) ist.

6. Medizinischer Behälter (1) nach dem vorhergehenden Anspruch, wobei die Antenne (42) eine Dipolantenne (42) ist, die zwei Schenkel (420) umfasst, die an einem Chip (40) des RFID-Tags (4) befestigt sind, wobei jeder der Schenkel (420) den mindestens einen Teil der Skala (14) bildet.

7. Medizinischer Behälter (1) nach dem vorhergehenden Anspruch, wobei sich einer der Schenkel (420) proximal zum Chip (40) erstreckt, während sich der andere Schenkel distal zum Chip (40) erstreckt.

8. Medizinischer Behälter (1) nach einem der vorhergehenden Ansprüche, wobei der RFID-Tag (4) einen Chip (40) einschließt, der nicht breiter als die Antenne (42) ist.

9. Medizinischer Behälter (1) nach einem der vorhergehenden Ansprüche, wobei die Antenne (42) eine beliebige von einer geraden Linien- oder einer Sinus-, einer Rechteck-, einer Dreieck-, einer Sägezahn- oder einer Impulswellenform aufweist.

10. Verfahren zur Herstellung eines medizinischen Behälters (1) nach einem der vorhergehenden Ansprüche, wobei das Verfahren die Schritte des Bildens einer RFID-Antenne (42) umfasst, die mindestens einen Teil der Skala (14) des Zylinders (10) bildet.

## Revendications

1. Récipient médical (1) comprenant un cylindre tubulaire (10) définissant un réservoir pour un produit médical, le cylindre (10) étant muni d'une échelle (14) ayant des graduations indicatives d'un volume injecté ou d'un volume restant dudit produit médical, le cylindre (10) comprenant en outre une étiquette RFID (4) située sur le cylindre (10), **caractérisé en ce que** l'étiquette RFID (4) comprend une antenne (42) qui forme au moins une partie de l'échelle (14).

2. Récipient médical (1) selon la revendication précédente, dans lequel l'étiquette RFID (4) comprend une puce (40), la puce (40) et l'antenne (42) étant appliquées sur un substrat transparent (60), où le substrat (60) est appliqué sur une paroi externe du cylindre (10).

3. Récipient médical (1) selon la revendication précédente, dans lequel le substrat (60) est muni d'un adhésif (66) pour la fixation de l'étiquette RFID (4) au cylindre (10).

4. Récipient médical (1) selon la revendication 1, dans lequel au moins l'antenne (42) de l'étiquette RFID (4) est formée par impression céramique métallique, impression métallique ou impression de graphène directement sur une paroi externe du cylindre (10).

5. Récipient médical (1) selon l'une des revendications précédentes, dans lequel l'étiquette RFID (4) est une étiquette RFID à ultra-haute fréquence (4).

6. Récipient médical (1) selon la revendication précédente, dans lequel l'antenne (42) est une antenne dipôle (42) comprenant deux branches (420) fixées à une puce (40) de l'étiquette RFID (4), chacune desdites branches (420) formant l'au moins une partie de l'échelle (14).

7. Récipient médical (1) selon la revendication précédente, dans lequel l'une desdites branches (420) s'étend de manière proximale par rapport à la puce (40) tandis que l'autre branche s'étend de manière distale par rapport à la puce (40).

8. Récipient médical (1) selon l'une des revendications précédentes, dans lequel l'étiquette RFID (4) comprend une puce (40) qui n'est pas plus large que l'antenne (42).

9. Récipient médical (1) selon l'une des revendications précédentes, dans lequel l'antenne (42) a l'une quelconque parmi une forme d'onde en ligne droite, sinusoïdale, carrée, triangulaire, en dents de scie ou d'impulsion.

10. Procédé de fabrication d'un récipient médical (1) selon l'une des revendications précédentes, dans lequel le procédé comprend les étapes de formation d'une antenne RFID (42) qui forme au moins une partie de l'échelle (14) du cylindre (10).
